# EUROPEAN PATENT APPLICATION

(11) **EP 4 556 484 A1**
(43) Date of publication of application: **21.05.2025**
(21) Application number: 23857740.7
(22) Date of filing: 24.08.2023
(51) Int. Cl.: C07K 14/37, C12N 15/70, A23K 20/147, C12N 9/24, A23L 33/18, D21H 17/00, D21H 17/22

(54) **VARIANT POLYPEPTIDE HAVING GLUCANASE ACTIVITY**

(30) Priority: 24.08.2022 KR 20220106311
(71) Applicant: CJ CheilJedang Corporation, Seoul 04560 (KR)
(72) Inventor: SON, Byung-sam, Seoul 04560 (KR); CHOI, Eun Jung, Seoul 04560 (KR)
(74) Representative: Meissner Bolte Partnerschaft mbB
(86) International application number: PCT/KR2023/012528
(87) International publication number: WO 2024/043708

(57) **Abstract**

The present application relates to a glucanase variant.

## Description

### [Technical Field]

The present application relates to a modified polypeptide having glucanase activity.

### [Background Art]

1,3-1,4-β-Glucan is a polysaccharide constituting the cell walls of various plants, and is particularly abundant in the cell walls of commercially valuable grains such as barley, rye, sorghum, rice, and wheat. However, glucan is an anti-nutritional factor that interferes with animals' digestion and absorption of plant-based feeds. Particularly in the case of monogastric animals, it is difficult to degrade and use glucan due to the absence of glucanase (EC 3.2.1.73), which can degrade such anti-nutritional factors.

Therefore, efficient nutrition intake of livestock may be possible by adding glucanase to feed, and for this purpose, development of a glucanase suitable for feed is required (Murphy P, Bello FD, O'Doherty JV, Arendt EK, Sweeney T, Coffey A (2012) Effects of cereal β-glucans and enzyme inclusion on the porcine gastrointestinal tract microbiota. Anaerobe 18(6):557-565).

### [Disclosure]

### [Technical Problem]

Meanwhile, thermal tolerance that can withstand high-temperature processes is essential in the case of enzymes for feeds.

### [Technical Solution]

It is one object of the present application to provide a modified polypeptide having glucanase activity, in which any one or more among the amino acids corresponding to positions 53, 199, and 200 from the N-terminus of SEQ ID NO: 1 is substituted with another amino acid.

It is another object of the present application to provide a polynucleotide encoding the polypeptide of the present application.

It is still another object of the present application to provide a microorganism, including one or more among the modified polypeptide of the present application; a polynucleotide encoding the modified polypeptide; and a vector containing the polynucleotide.

It is yet another object of the present application to provide a composition for degrading glucan, including one or more among the polypeptide of the present application and the microorganism of the present application.

It is even another object of the present application to provide a method for degrading glucan, including: reacting one or more among the polypeptide of the present application and the microorganism of the present application with glucan.

It is further another object of the present application to provide a feed additive composition, including one or more among the polypeptide of the present application and the microorganism of the present application.

It is still further another object of the present application to provide a method for preparing feed product, including: mixing a feed additive composition including one or more among the polypeptide of the present application and the microorganism of the present application with a feed component.

It is still further another object of the present application to provide a food composition; a composition for preparing food; a detergent composition; a fabric composition; and a paper processing composition, including one or more among the polypeptide of the present application and the microorganism of the present application.

It is still further another object of the present application to provide a method for preparing a modified polypeptide having glucanase activity, including: culturing the microorganism of the present application.

### [Advantageous Effects]

The modified polypeptide having glucanase activity of the present application has improved thermal tolerance and thus can be advantageously applied in various industrial fields.

### [Brief Description of the Drawing]

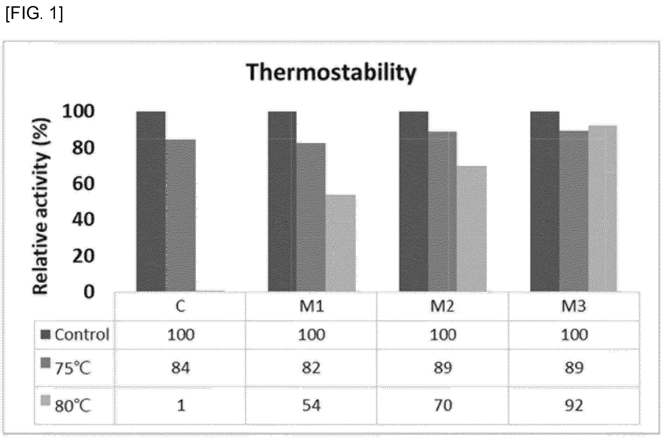

FIG. 1 is a result of evaluating the thermal tolerance of C, M1, M2, and M3 enzymes.

### [Detailed Description of Preferred Embodiments]

Hereinafter, the present application will be described in detail. Meanwhile, each description and embodiment disclosed herein can be applied to other descriptions and embodiments, respectively. That is, all combinations of various elements disclosed herein fall within the scope of the present application. Further, the scope of the present application is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present application.

Further, a number of papers and patent documents have been cited throughout the present specification. The content of the cited papers and patent documents is incorporated herein by reference in their entirety and the level of technical field to which the present invention belongs and the contents of the present invention will be described more clearly.

One aspect of the present application provides a modified polypeptide having glucanase activity, in which any one or more among the amino acids corresponding to positions 53, 199, and 200 from the N-terminus of SEQ ID NO: 1 is substituted with another amino acid.

As used herein, the term "modified polypeptide having glucanase activity" means a variant including substitutions of any one or more amino acids among the amino acids corresponding to positions 53, 199, and 200 from the N-terminus of SEQ ID NO: 1 with another amino acid, in any polypeptide having glucanase activity or glucanase.

As used herein, the term "glucanase" means an enzyme having an activity capable of cleaving a glucosidic bond connecting two glucosyl residues in glucan. Non-limiting examples of glucanase activity include endo-cellulase activity for *beta-*1,4 linkages between D-glucoses, and licheninase or lichenase activity that cleaves (1,4)-*beta*-D-glucosidic bonds of *beta*-D-glucans containing 1,3- and 1,4-linkages. As one embodiment, the glucanase of the present application may have an activity corresponding to glucanase classified as EC 3.2.1.73.

In one embodiment, the reference glucanase targeted for mutation in the present application may be a protein having the amino acid sequence of SEQ ID NO: 1 or a protein including an amino acid sequence having a homology or identity of at least 70%, 75%, 80%, 85%, 90%, 95%, 96%, 97%, 98% or more, or 99% or more to the amino acid sequence of SEQ ID NO: 1. Additionally, it is apparent that any protein having an amino acid sequence, in which part of the sequence is deleted, modified, substituted, or added, may also fall within the scope of the reference protein targeted for mutation of the present application as long as the amino acid sequence has such a homology or identity and exhibits an efficacy corresponding to that of the protein of the present application.

Although the protein including the amino acid sequence of SEQ ID NO: 1 has been defined as an example of the reference glucanase, it does not exclude an addition of a meaningless sequence upstream or downstream of the amino acid sequence, a mutation that may occur naturally, or a silent mutation thereof, and it is apparent to those skilled in the art that any protein having an activity identical or corresponding to the protein consisting the amino acid sequence of SEQ ID NO: 1 may fall within the scope of the reference glucanase. Examples of other reference glucanases include a glucanase derived from *Orpinomyces* sp., *Neocallimastix* sp., *Piromyces* sp., *Clostridium* sp., *Rhodothermus* sp., or *Ruminococcus* sp. An example of still another glucanase includes a wild-type glucanase derived from *Orpinomyces* sp.

As used herein, the term "variant" or "modified polypeptide" refers to a protein having one or more amino acids different from the recited sequence by conservative substitutions and/or modifications such that the functions and properties of the protein are retained.

The variants are different from the sequences identified by substitution, deletion, or addition of several amino acids. Such variants may generally be identified by modifying one or more of the above amino acid sequences of the protein and evaluating the properties of the modified protein. That is, the ability of the variants may be enhanced, unchanged or reduced relative to a native protein. Further, some variants may include those in which one or more regions, such as an N-terminal leader sequence or transmembrane domain, have been removed. Further, other variants may include those in which a region has been removed from the N- and/or C-terminus of a mature protein. The term "variant" or "modified polypeptide" may be used interchangeably with terms such as modification, modified protein, mutant, mutein, divergent, variant, *etc.* without limitation, as long as the terms are used to indicate variation.

As used herein, the term "conservative substitution" refers to substitution of an amino acid with another amino acid having similar structural and/or chemical properties. Typically, conservative substitutions may have little or no effect on the activity of the resulting polypeptide.

The variant may have, for example, one or more conservative substitutions while still retaining one or more biological activities. Such amino acid substitution may generally occur based on similarity of polarity, charge, solubility, hydrophobicity, hydrophilicity, and/or amphipathic nature of a residue.

The amino acids may be classified into the following groups:
In one example, positively charged (basic) amino acids include arginine, lysine, and histidine; negatively charged (acidic) amino acids include glutamic acid and aspartic acid; amino acids having nonpolar side chains (nonpolar amino acids) include glycine, alanine, valine, leucine, isoleucine, methionine, phenylalanine, tryptophan, and proline; amino acids having polar or hydrophilic side chains (polar amino acids) include serine, threonine, cysteine, tyrosine, asparagine, and glutamine. In another example, amino acids having electrically charged side chain include arginine, lysine, histidine, glutamic acid, aspartate; and amino acids having uncharged amino acids (referred to as neutral amino acids) include glycine, alanine, valine, leucine, isoleucine, methionine, phenylalanine, tryptophan, proline, serine, threonine, cysteine, tyrosine, asparagine, and glutamine. In still another example, aromatic amino acids include phenylalanine, tryptophan, and tyrosine. In yet another example, branched amino acids include valine, leucine, and isoleucine. In even another example, the 20 amino acids can be classified according to their size into 5 groups, starting from amino acid groups with a relatively small volume, i.e., glycine, alanine, serine; cysteine, proline, threonine, aspartate, asparagine; valine, histidine, glutamic acid, glutamine; isoleucine, leucine, methionine, lysine, arginine; and phenylalanine, tryptophan, and tyrosine, but the classification of the amino acids is not limited thereto.

Additionally, the variant may also include deletion or addition of amino acids that have minimal influence on the properties and secondary structure of a polypeptide. For example, the polypeptide may be conjugated with a signal (or leader) sequence at the N-terminus involved in the transfer of proteins co-translationally or post-translationally. Further, the polypeptide may also be conjugated with another sequence or linker to identify, purify, or synthesize the polypeptide.

In one embodiment, the modified polypeptide of the present application may be a modified polypeptide having glucanase activity, in which any one or more among the amino acids corresponding to positions 53, 199, and 200 from the N-terminus of SEQ ID NO: 1 is substituted with another amino acid, among the above-described glucanases.

In the present application, the "substitution with another amino acid" is not limited as long as it is substituted with an amino acid other than the amino acid before substitution. Meanwhile, in the present application, when it is expressed that "a specific amino acid has been substituted", it is apparent that the amino acid is substituted with an amino acid different from the amino acid before substitution, even if it is not specifically stated that the amino acid has been substituted with a different amino acid.

In any one of the embodiments described above, the modified polypeptide of the present application may include substitution of the amino acid corresponding to position 53 with cysteine (C).

In any one of the embodiments described above, the modified polypeptide of the present application may include substitution of the amino acid corresponding to position 199 with cysteine (C).

In any one of the embodiments described above, the modified polypeptide of the present application may include substitution of the amino acid corresponding to position 200 with proline (P).

In any one of the embodiments described above, the modified polypeptide of the present application may include any one or more substitutions from the following:
i) substitution of the amino acid corresponding to position 53 with cysteine (C);
ii) substitution of the amino acid corresponding to position 199 with cysteine (C); and
iii) substitution of the amino acid corresponding to position 200 with proline (P).

In any one of the embodiments described above, the modified polypeptide of the present application may include substitutions selected from the following:
(i) substitution of the amino acid corresponding to position 200 with proline (P);
(ii) substitution of the amino acid corresponding to position 53 with cysteine (C) and substitution of the amino acid corresponding to position 199 with cysteine (C); or
(iii) substitution of the amino acid corresponding to position 200 with proline (P), substitution of the amino acid corresponding to position 53 with cysteine (C), and substitution of the amino acid corresponding to position 199 with cysteine (C).

In any one of the embodiments described above, the modified polypeptide of the present application may include substitution of the amino acid corresponding to position 53 with cysteine (C) and substitution of the amino acid corresponding to position 199 with cysteine (C), and modification of the amino acid pair (i.e., between cysteine residues) to form a disulfide bond.

The "N^{th} position" of the present application may include the N^{th} position and an amino acid position corresponding to the N^{th} position. For example, it may include an amino acid position corresponding to any amino acid residue in a mature polypeptide disclosed in a specific amino acid sequence. In one example, the specific amino acid sequence may be the amino acid sequence of SEQ ID NO: 1.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the "corresponding region" generally refers to a similar or corresponding position in the related protein or reference protein.

In the present application, a specific numbering of amino acid residue positions in the protein used herein may be employed. For example, it is possible to renumber the amino acid residue positions of the protein of the present application to the corresponding positions by aligning the sequence of the protein of the present application with the target protein to be compared.

For example, any amino acid sequence is aligned with SEQ ID NO: 1, and based on the alignment, each amino acid residue of the amino acid sequence can be numbered with reference to the numerical position of the amino acid residue corresponding to the amino acid residue of SEQ ID NO: 1. For example, a sequence alignment algorithm such as that described herein can identify the position of an amino acid or a position where modifications such as substitutions, insertions or deletions occur compared to a query sequence (also referred to as a "reference sequence").

Example of the alignment may be determined by the Needleman-Wunsch algorithm (Needleman and Wunsch, 1970, J. Mol. Biol. 48:443-453) and Needleman program of the EMBOSS package (EMBOSS: The European Molecular Biology Open Software Suite, Rice et al., 2000, Trends Genet. 16:276-277), *etc.,* but is not limited thereto, and sequence alignment programs, such as pairwise sequence comparison algorithms, *etc.*, known in the art may be appropriately used.

In one embodiment, the modified polypeptide of the present application may be one in which any one or more among the amino acids corresponding to positions 53, 199 and 200 from the N-terminus of SEQ ID NO: 1 is substituted with another amino acid and include the amino acid sequence of SEQ ID NO: 1 or an amino acid sequence having a homology or identity of at least 80%, 85%, 90%, 95%, 96%, 97%, 98%, or 99% or more to the amino acid sequence of SEQ ID NO: 1.

In any one of the embodiments described above, the modified polypeptide of the present application may be one in which any one or more among the amino acids corresponding to positions 53, 199, and 200 from the N-terminus of SEQ ID NO: 1 is substituted with another amino acid and include the amino acid sequence of SEQ ID NO: 1 or have a homology or identity of at least 80%, 85%, 90%, 95%, 96%, 97%, 98%, or 99% or more to the amino acid sequence of SEQ ID NO: 1.

In any one of the embodiments described above, the modified polypeptide of the present application may include any one of amino acid sequences of SEQ ID NOS: 2 to 4.

In any one of the embodiments described above, the modified polypeptide of the present application may consist essentially of any one of amino acid sequences of SEQ ID NOS: 2 to 4, and more specifically consist of any one of amino acid sequences of SEQ ID NOS: 2 to 4, but is not limited thereto.

In any one of the embodiments described above, the variant may include any one of amino acid sequences of SEQ ID NOS: 2 to 4, or a sequence having a homology or identity of at least 80% or more thereto while the amino acids corresponding to positions 53, 199, and 200 in the amino acid sequence are fixed (i.e., in the amino acid sequence of the variant, the amino acids corresponding to positions 53, 199, and 200 of any one of amino acid sequences of SEQ ID NOS: 2 to 4 are the same as the amino acids at positions 53, 199, and 200 of any one of amino acid sequences of SEQ ID NOS: 2 to 4).

In addition, it is apparent that any protein having an amino acid sequence, in which part of the sequence is deleted, modified, substituted, or added, may also fall within the scope of the present application as long as the amino acid sequence has such a homology or identity and exhibits an effect corresponding to that of the variant.

As used herein, the term "homology" or "identity" refers to a degree of similarity between two given amino acid sequences or nucleotide sequences, and may be expressed as a percentage. The terms "homology" and "identity" may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or highly stringent conditions. It is apparent that hybridization with polynucleotides containing general codon or degenerate codons in hybridizing polynucleotides is also included.

Whether any two polynucleotide or polypeptide sequences have a homology, similarity, or identity may be, for example, determined by a known computer algorithm such as the "FASTA" program (Pearson et al., (1988) Proc. Natl. Acad. Sci. USA 85:2444) using default parameters. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (preferably, version 5.0.0 or later) (GCG program package (Devereux J et al., Nucleic Acids Research 12:387 (1984)), BLASTP, BLASTN, FASTA (Atschul SF et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity, or identity as the value obtained by dividing the number of similarly aligned symbols (*i.e.*, nucleotides or amino acids) by the total number of the symbols in the shorter of the two sequences. Default parameters for the GAP program may include (1) a unitary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Therefore, the term "homology" or "identity" used herein indicates relatedness between sequences.

As used herein, the "enzymatic activity" or "glucanase activity" exhibits at least one catalytic activity. Specifically, it may be the conversion efficiency of an enzyme mainly expressed as k_{cat}/Kₘ, but is not limited thereto.

When the enzyme is completely saturated with substrates, k_{cat} means the rate constant (catalytic constant) that converts substrates into products in unit time by one enzyme, and is also referred to as a turnover number. Kₘ is the substrate concentration when the reaction rate is at half the maximum value (Vₘₐₓ).

Examples of the ways to express enzymatic activity include specific activity (µmol of converted substrate × mg⁻¹ × min⁻¹) or volumetric activity (µmol of converted substrate × mL⁻¹ × min⁻¹), *etc.* However, defining the enzymatic activity is not limited to the contents described above, and the enzymatic activity can be defined and evaluated based on the information known in the art.

As used herein, enzyme stability means that enzyme activity is maintained during storage or reaction time. In order to measure such changes in stability, the initial enzyme activity can be measured and compared under defined conditions at time zero (100%) and after a certain period of time (x%), and thus, the level at which the enzyme activity is lost or enzyme stability can be expressed.

Factors that affect enzyme activity include, for example, pH, heat, the presence of other substances (e.g., oxidizing agents, chelating agents), *etc.*

As used herein, the term "thermal stability" means the ability of a protein to function in a specific temperature range. In one embodiment, the variant provided in the present application may have activity in the range of about 20°C°C to about 120°C, and specifically may have activity in the range of about 60°C to about 100°C, but is not limited thereto.

As used herein, the term "thermal tolerance" means the ability of a protein to function after exposure to a specific temperature, e.g., high heat or cryogenic temperature. For example, proteins that are thermotolerant may not function at a temperature they are exposed to, but may become functional when returned to an optimal temperature environment. In one embodiment, the modified polypeptide provided in the present application can maintain the ability to function after being exposed to high temperatures in the range of about 60°C to about 100°C.

An increase in stability or thermal tolerance may include maintaining high enzymatic activity; increasing the range of pH, temperature and/or time, *etc.,* at which a protein maintains its function, by comparing to other enzymes, e.g., a wild-type enzyme, parent enzyme and/or other variants.

In one embodiment, the modified polypeptide provided in the present application may have improved thermal tolerance and/or thermal stability compared to the polypeptide of SEQ ID NO: 1.

In one embodiment, the modified polypeptide provided in the present application may have improved thermal tolerance and/or thermal stability compared to a wild-type polypeptide.

In one embodiment, the modified polypeptide provided in the present application may have improved thermal tolerance compared to the polypeptide of SEQ ID NO: 1.

In any one of the embodiments described above, the properties exhibited by the modified polypeptide provided in the present application activity may be suitable or improved activity for application in various industrial fields, including feed, baking, pulp bleaching, *etc.*

Another aspect of the present application provides a polynucleotide encoding the modified polypeptide of the present application.

As used herein, the "polynucleotide", which is a polymer of nucleotides composed of nucleotide monomers connected in a lengthy chain by a covalently bond, is a DNA or RNA strand having at least a certain length.

The polynucleotide encoding the modified polypeptide of the present application may include any polynucleotide encoding the modified polypeptide of the present application or a polypeptide having activity corresponding to the modified polypeptide, without limitation.

The polynucleotide of the present application may undergo various modifications in the coding region within the scope that does not change the amino acid sequence of the polypeptide, due to codon degeneracy or in consideration of the codons preferred in an organism in which the polypeptide is to be expressed.

In one embodiment, the polynucleotide encoding the modified polypeptide of the present application may have or include a nucleotide sequence having a homology or identity of at least 50%, 60%, 70%, 75%, 80%, 85%, or 90% or more, and 100% or less with the polynucleotide sequence of SEQ ID NO: 5.

In any one of the embodiments described above, the polynucleotide encoding the glucanase variant of the present application may have or include a nucleotide sequence having a homology or identity of at least 50%, 60%, 70%, 75%, 80%, 85%, or 90% or more, and 100% or less with the polynucleotide sequence of SEQ ID NO: 5, and in the sequence having such homology or identity, the codon encoding the amino acid corresponding to position 53 from the N-terminus of SEQ ID NO: 1 may be one of the codons encoding amino acids other than arginine (R); the codon encoding the amino acid corresponding to position 199 may be one of the codons encoding amino acids other than threonine (T); the codon encoding the amino acid corresponding to position 200 may be one of the codons encoding amino acids other than asparagine (N); or a combination thereof.

In any one of the embodiments described above, the polynucleotide encoding the glucanase variant of the present application may have or include a nucleotide sequence having a homology or identity of at least 50%, 60%, 70%, 75%, 80%, 85%, or 90% or more, and 100% or less with the polynucleotide sequence of SEQ ID NO: 5, and in the sequence having such homology or identity, the codon encoding the amino acid corresponding to position 53 and/or 199 from the N-terminus of SEQ ID NO: 1 may be one of the codons encoding cysteine (C), and/or the codon encoding the amino acid corresponding to position 200 may be one of the codons encoding proline (P).

Additionally, the polynucleotide of the present application may include a probe that may be prepared from a known gene sequence, for example, any sequence which can hybridize with a sequence complementary to all or part of the polynucleotide sequence of the present application under stringent conditions without limitation.

The "stringent conditions" refers to conditions under which specific hybridization between polynucleotides is allowed. Such conditions are specifically described in the literature (J Sambrook et al., Molecular Cloning, A Laboratory Manual, 2nd Edition, Cold Spring Harbor Laboratory press, Cold Spring Harbor, New York, 1989; FM Ausubel et al., Current Protocols in Molecular Biology, John Wiley & Sons, Inc., New York, 9.50-9.51, 11.7-11.8). For example, the stringent conditions may include conditions under which genes having a high homology or identity of 70% or more, 75% or more, 80% or more, 85% or more, 90% or more, 95% or more, 96% or more, 97% or more, 98% or more, or 99% or more are hybridized with each other and genes having a homology or identity lower than the above homologies or identities are not hybridized with each other, or washing conditions of Southern hybridization, that is, washing once, specifically, twice or three times at a salt concentration and a temperature corresponding to 60°C, 1 ×SSC, 0.1% SDS, specifically 60°C, 0.1 xSSC, 0.1% SDS, and more specifically 68°C, 0.1×SSC, 0.1% SDS.

Hybridization requires that two nucleic acids contain complementary sequences, although mismatches between bases are possible depending on the stringency of the hybridization. The term "complementary" is used to describe the relationship between nucleotide bases that can hybridize with each other. For example, with respect to DNA, adenine is complementary to thymine, and cytosine is complementary to guanine. Therefore, the polynucleotide of the present application may include isolated nucleotide fragments complementary to the entire sequence as well as nucleic acid sequences substantially similar thereto.

Specifically, polynucleotides having a homology or identity with the polynucleotide of the present application may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e.g.*, Sambrook *et al.*)*.*

Still another aspect of the present application provides a vector containing a polynucleotide encoding the modified polypeptide of the present application. The modified peptide and the polynucleotide are as described in other aspects above.

As used herein, the term "vector" may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable microorganism, the vector may replicate or function independently from the host genome, or may integrate into genome thereof.

In one example, a polynucleotide encoding a target protein may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

The vector used in the present application is not particularly limited, and any vector known in the art may be used.

Examples of the vector typically used in prokaryotic cells may include, as a phage vector or cosmid vector, pWE15, M13, MBL3, MBL4, IXII, ASHII, APII, t10, t11, Charon4A, and Charon21A, *etc.*; and as a plasmid vector, those based on pBR, pUC, pBluescriptII, pGEM, pTZ, pCL and pET, *etc.* Specifically, pDZ, pACYC177, pACYC184, pCL, pECCG117, pUC19, pBR322, pMW118, pCC1BAC, pDCM2 vector, *etc.* may be used.

As examples of the vector used in eukaryotic cells, yeast expression vector may include both yeast integrative plasmid (YIp) and extrachromosomal plasmid vector. The extrachromosomal plasmid vector may include yeast episomal plasmid (YEp), yeast replicative plasmids (YRp), and yeast centromere plasmid (YCp). In addition, yeast artificial chromosomes (YACs) can also be used as the vector of the present application. Specific examples of the vector that can be used may include pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405 and pYJ406, but are not limited thereto.

As used herein, the term "transformation" refers to the introduction of a vector containing a polynucleotide encoding a target protein into a host cell or a microorganism so that the protein encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target protein. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but is not limited thereto.

Further, as used herein, the term "operably linked" means that the gene sequence is functionally linked to a promoter sequence that initiates and mediates transcription of the polynucleotide encoding the target polypeptide of the present application.

The method of transforming the vector of the present application may include any method which can introduce a nucleic acid into a cell, and the transformation may be performed by selecting an appropriate standard technique as known in the art according to the host cell. For example, the method may include electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate-DMSO method, *etc.,* but is not limited thereto.

Yet another aspect of the present application provides a microorganism including one or more among the modified polypeptide of the present application; a polynucleotide encoding the modified polypeptide; and a vector containing the polynucleotide.

The microorganism may include the above-described modified polypeptide, a polynucleotide encoding the variant, a nucleic acid construct and/or vector containing the polynucleotide. The nucleic acid construct or vector may be integrated into the chromosome, or may remain as a self-replicating extrachromosomal vector.

The microorganism of the present application may include any microorganism as long as it can express the modified polypeptide of the present application without limitation. For example, the microorganism may include any cell useful in recombinant production of the modified polypeptide of the present application, e.g., a prokaryote or a eukaryote. In another example, the microorganism includes fungi and bacteria.

In one embodiment, the microorganism of the present application may be a microorganism belonging to the genus *Escherichia*, and may be *Escherichia coli*, *Escherichia albertii, Escherichia fergusonii, Escherichia hermannii, Escherichia vulneris*, or *Escherichia blattae.* In any one of the embodiments described above, the microorganism may be *Escherichia coli*, but is not limited thereto.

Even another aspect of the present application provides a composition for degrading glucan, including one or more among the modified polypeptide of the present application; and a microorganism including one or more among the modified polypeptide, a polynucleotide encoding the modified polypeptide, and a vector containing the polynucleotide.

Even another aspect of the present application provides a use of a composition for degrading glucan, wherein the composition includes one or more among the modified polypeptide of the present application; and a microorganism including one or more among the modified polypeptide, a polynucleotide encoding the modified polypeptide, and a vector containing the polynucleotide.

Further another aspect of the present application provides a method for degrading glucan, including: reacting one or more among the modified polypeptide of the present application; and a microorganism including one or more among the modified polypeptide, a polynucleotide encoding the modified polypeptide, and a vector containing the polynucleotide with glucan.

The modified polypeptide of the present application and/or the microorganism including one or more among the modified polypeptide, a polynucleotide encoding the modified polypeptide, and a vector containing the polynucleotide may be used to degrade a glucan-containing material.

In one example, the composition for degrading glucan provided in the present application may further include a naturally occurring material or a non-naturally occurring material, in addition to the modified polypeptide of the present application.

In one embodiment, the composition for degrading glucan provided in the present application may further include any constituting element suitable for application in various industrial fields, including animal feed, baking, biomass saccharification, pulp bleaching, *etc.*

Examples of materials that may be added include stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, diluents, lubricants, *etc.,* but are not limited thereto.

Still further another aspect of the present application provides a feed additive composition, including one or more among the modified polypeptide of the present application; and a microorganism including one or more among the modified polypeptide, a polynucleotide encoding the modified polypeptide, and a vector containing the polynucleotide.

Still further another aspect of the present application provides a method for preparing feed products, including: mixing a feed additive composition including one or more among the modified polypeptide of the present application; and a microorganism including one or more among the modified polypeptide, a polynucleotide encoding the modified polypeptide, and a vector containing the polynucleotide with a feed component.

The modified polypeptide of the present application or the microorganism of the present invention may be used in any one of the following applications:
a) an additive in animal feedstuffs; and/or
b) a feed supplement for animals; and/or
c) breakdown of grain-based materials (*e.g.*, this can be whole grain or part of grain).

The feed product of the present application may refer to any natural or artificial diet, a single meal, *etc.,* or a component of the single meal, which an animal eats, ingests and digests, or which is suitable for eating, ingestion and digestion, and the feed product may be prepared in various forms known in the art.

The modified polypeptide having glucanase activity of the present application can be used in various industrial fields such as food, paper, pulp, detergent, industrial waste treatment, biomaterials and bioenergy, *etc.* With respect to the application of glucanase in the industrial fields, literature such as McCarthy TC, Lalor E, Hanniffy O, Savage AV, Tuohy MG, Comparison of wide-type and UV-mutant βglucanase-producing strains of Talaromyces emersonii with potential in brewing applications, J Ind Microbiol Biotechnol, 2005; Chaari F, Belghith-Fendri L, Blibech M, Driss D, Ellouzia SZ, Sameh M, Ellouz-Chaabouni S, Biochemical characterization of a lichenase from Penicillium occitanis Pol6 and its potential application in the brewing industry, Process Biochem, 2014; Maktouf S, Moulis C, Kamoun A, Chaari F, Chaabouni SE, Remaud-Simeon M, A laundry detergent compatible lichenase: statistical optimization for production under solid state fermentation on crude millet, Ind Crops Prod, 2013; *etc.* may be incorporated by reference.

Still further another aspect of the present application provides a food composition; or a composition for preparing food, including the modified polypeptide having glucanase activity of the present application or the microorganism of the present application. In one example, glucanase can be used for brewing (beer, wine, *etc.*) and for producing fruit juice. Glucanase can be added during saccharification to reduce viscosity of wort, improve filtration performance, increase the dissolution rate of malt, prevent beer turbidity, and stabilize quality of beer.

Still further another aspect of the present application provides a detergent composition including the modified polypeptide having glucanase activity of the present application or the microorganism of the present application. Detergent compositions are compositions used to remove unwanted compounds from items to be cleaned, such as fabrics, dishes, or hard surfaces. Examples of detergent compositions include fabric detergents, textile detergents, hard surface cleaners for glass, wood, plastic, ceramic, metal countertops and windows, *etc.,* carpet cleaners, oven cleaners, fabric fragrances, fabric softeners, dishwasher detergents, *etc.*

Still further another aspect of the present application provides a fabric composition including the modified polypeptide having glucanase activity of the present application or the microorganism of the present application. The glucanases of the present application can be used in the processing of fibers for the manufacture of garments. These include processes such as bleaching, dye removal after dyeing, and biopolishing of cotton fabrics or cotton blends.

Still further another aspect of the present application provides a paper processing composition including the modified polypeptide having glucanase activity of the present application or the microorganism of the present application. The modified polypeptide having glucanase activity of the present application can be used for improving wastewater, removing ink or decolorization in the pulp industry.

In any one of the embodiments described above, the composition provided in the present application may further include any constituting element suitable for application in various industrial fields. In any one of the embodiments described above, the composition provided in this application may further include any other enzyme suitable for the intended use.

In any one of the embodiments described above, the composition provided in the present application may include the modified polypeptide having glucanase activity of the present application or the microorganism of the present application in an amount suitable for the intended use.

In any one of the embodiments described above, the composition provided in the present application may be provided in a form suitable for the intended use. As non-limiting examples, the composition may be in the form of bars, tablets, powder, granules, pastes or liquid.

Still further another aspect of the present application provides a method for preparing a modified polypeptide having glucanase activity, including: culturing a microorganism including one or more among the modified polypeptide, a polynucleotide encoding the modified polypeptide, and a vector containing the polynucleotide.

The cultivation may include culturing the microorganism in a culture medium.

As used herein, the term "cultivation" means that the host cell is grown under appropriately controlled environmental conditions. The cultivation process of the present application may be performed in a suitable culture medium and culture conditions known in the art. Such a cultivation process may be easily adjusted for use by those skilled in the art according to the strain to be selected. Specifically, the cultivation may be a batch culture, a continuous culture, and a fed-batch culture, but is not limited thereto.

As used herein, the term "medium" refers to a mixture of materials which contains nutrient materials required for the cultivation of the host cell as a main ingredient, and it supplies nutrient materials and growth factors, along with water that is essential for survival and growth. Specifically, the medium and other culture conditions used for culturing the host cell of the present application may be any medium used for conventional cultivation of host cells without any particular limitation. However, the host cell of the present application may be cultured under aerobic conditions in a conventional medium containing an appropriate carbon source, nitrogen source, phosphorus source, inorganic compound, amino acid, and/or vitamin, *etc.,* while adjusting temperature, pH, *etc.*

In the present application, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.*, molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the host cell in an appropriate manner. In addition, bubble formation may be prevented during the cultivation using an antifoaming agent such as fatty acid polyglycol ester. Further, oxygen gas or a gas containing oxygen may be injected to the medium in order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature of the medium may be in the range from 20°C to 55°C, specifically from 25°C to 40°C, but is not limited thereto. The cultivation may be continued until the desired amount of a useful material is obtained, and may be specifically carried out for 24 to 196 hours, but is not limited thereto.

In one embodiment, the method for preparing the modified polypeptide of the present application may further include recovering the modified polypeptide of the present application expressed in the culturing step.

In another embodiment, the modified polypeptide expressed in the culturing step may be recovered using methods known in the art to which the present application pertains. For example, the modified polypeptide may be recovered from a medium by conventional procedures including, but not limited to, collection, centrifugation, filtration, extraction, spray-drying, evaporation, or precipitation.

With respect to the recovering method, the modified polypeptide may be collected using the method of culturing the microorganism of the present application, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC and a combination thereof may be used, and the variant of the present application may be recovered from the medium or the host cell using suitable methods known in the art.

In another embodiment, the modified polypeptide expressed by the host cell in the culturing step may not be recovered. In the embodiment, the host cell expressing the modified polypeptide may be used as a source of the modified polypeptide.

Still another aspect of the present application provides a composition including one or more among the modified polypeptide of the present application; and a microorganism including one or more among the modified polypeptide, a polynucleotide encoding the modified polypeptide, and a vector containing the polynucleotide.

The composition can be used for food, feed additive, food preparation, detergent, fabric or paper processing as described above. Still further another aspect of the present application provides a use of the composition for food, feed additive, food preparation, detergent, fabric or paper processing, as described in other aspects above.

### [Mode for Carrying Out the Invention]

Hereinafter, the present application will be described in more detail by way of Examples and Experimental Examples. However, these Examples and Experimental Examples are given for illustrative purposes only, and the scope of the present application is not intended to be limited by these Examples and Experimental Examples.

### Example 1. Preparation of Protein Having Glucanase Activity

### Example 1-1. Preparation of Variant Recombinant Plasmids

Based on the synthetic sequence C (SEQ ID NO: 1) prepared from the gene derived from the genomic DNA of the PC-2 strain belonging to the genus *Orpinomyces*, three variants, M1 (SEQ ID NO: 2), M2 (SEQ ID NO: 3), and M3 (SEQ ID NO: 4), were prepared.

PCR was performed under the following conditions to amplify variant genes for cloning. Specifically, the variants were prepared by PCR using the template, primers, and a PCR premix (Taq 2X premix, Bioneer). M1 was prepared using M1-F and M1,R primer based on the synthetic sequence C, M2 was prepared using M2-F1, M2-F2, M2-R1, and M2-R2 primers based on the synthetic sequence C, and M3 was prepared using M3-F and M3R primer based on M2.

PCR was performed using Eppendorf Mastercycler Nexus GX2, and the reaction conditions are as follows:
Initial Denaturation: 95°C, 2 min
Denaturation: 95°C, 20 sec
Annealing: 50°C, 10 sec
Extension: 72°C, 1 min (30 cycles from Denaturation to Extension)
Final Extension: 72°C, 5 min

The amplified genes were cloned into the pET28a vector using an In-Fusion HD cloning kit (Takara, Cat. No. 639650).

**[Table 1]**

| | **Primer** | | **Sequence** |
|---|---|---|---|
| | >M1-F | AACGGCCGTACCCCGATTAGCGCGTA T | |
| | >M1-R | ATACGCGCTAATCGGGGTACGGCCGT T | |
| | >M2-F1 | TTGACCATTGACTGCGATGGCAGCGG T | |
| | >M2-F2 | TTTAACGGCCGTTGCAATATTAGCGC G | |
| | >M2-R1 | ACCGCTGCCATCGCAGTCAATGGTCA A | |
| | >M2-R2 | CGCGCTAATATTGCAACGGCCGTTAA A | |
| | >M3-F | AACGGCCGTTGCCCGATTAGCGCGTA T | |
| | >M3-R | ATACGCGCTAATCGGGCAACGGCCGT T | |

M1, M2, and M3 variants were mutated at the following sites: In M1, asparagine, the amino acid at position 200 in SEQ ID NO: 1, is substituted with proline. In M2, arginine at position 53 in SEQ ID NO: 1 was substituted with cysteine, and threonine at position 199 was substituted with cysteine. In M3, in SEQ ID NO: 1, arginine at position 53 was substituted with cysteine, threonine at position 199 was substituted with cysteine, and asparagine at position 200 was substituted with proline.

### Example 1-2. Preparation of Transformant Having Glucanase Gene and Enzyme Expression

The recombinant plasmids prepared in Example 1-1 was transformed into *E. coli* BL21. In addition, in order to produce glucanase variants, the transformants were inoculated into 5 mL of LB medium and incubated at 37°C for 18 hours. For the main culture, 5 mL of the pre-culture medium was inoculated into 300 mL of LB medium and incubated at 37°C. Then, when the OD₆₀₀ value reached between 0.4-0.6, 120 µL of 1 M IPTG was added and incubated for an additional 12 hours. Cells were recovered through centrifugation and redispersed in 35 mL of lysis buffer (50 mM Tris-HCl pH 8.0, 100 mM NaCl, 10 mM imidazole).

### Example 1-3. Purification of Polypeptides Having Glucanase Activity

The cells in the dispersed solution were disrupted using an ultrasonic disperser, and a crude enzyme solution, the supernatant, was obtained through centrifugation. The crude enzyme solution was flowed through a Ni-NTA resin (Qiagen, Cat. No. 30230) to be adsorbed, followed by sequentially flowing washing buffer (only 20 mM of imidazole in the lysis buffer composition) and elution buffer (only 250 mM of imidazole in the lysis buffer composition) to obtain purified polypeptides having glucanase activity.

### Example 2. Evaluation of Thermal Tolerance of Glucanase Variants

### Example 2-1. Measurement of Residual Activity of Glucanase Variants

In order to evaluate the thermal tolerance of the variants, each purified enzyme was allowed to stand at 75°C and 80°C for 5 minutes. In order to measure the activity of the heat-treated enzymes, 50 µL of 1% glucan, 5 µL of 1 M buffer, and an appropriate amount of the enzymes before and after heat treatment were mixed, respectively, and tertiary distilled water was added to a final volume of 100 µL. Thereafter, the mixture was well stirred and reacted at 37°C, and the produced reducing sugar was measured by the DNS method. The method for preparing a DNS solution was as follows:
6.3g of 3,5-dinitrosalicylic acid (samchus, D1267) was added to 500ml of distilled water and dissolved at 50°C while 21g of Sodium Hydroxide (Daejung, 7571-4400) was added. Then, 182g of Potassium sodium tartrate tetrahydrate (Daejung, 6618-4400) and 5g of Phenol (Sigma-Aldrich, P1037) were added, and 300ml of distilled water was added. After adding 5g of Sodium sulfite anhydrous (Daejung, 7634-4405), stirring to be dissolved and cooling, the final volume was adjusted to 1000ml with distilled water and then filtered to complete the preparation.

### Example 3. Results of Thermal Tolerance of Variants

In order to evaluate the thermal tolerance of proteins and variants having glucanase activity, the purified enzymes C, M1, M2, and M3 prepared in Examples 1-3 were each allowed to stand at 75°C and 80°C for 5 minutes. Then, the residual activity was confirmed at 37°C using the heat-treated enzymes.

As a result, all three variants (M1, M2, M3) showed improved thermal tolerance compared to C. When the enzyme activity at 37°C before heat treatment was set to 100%, the thermal tolerance of M1 was found to be 54%, and the thermal tolerance of M2 was found to be 70%, which were improved by more than 50% relative to 1% of C, under the temperature condition of 80°C. Additionally, M3 showed excellent residual activity of 92% or more.

From the foregoing, a skilled person in the art to which the present application pertains will be able to understand that the present application may be embodied in other specific forms without modifying the technical concepts or essential characteristics of the present application. In this regard, the exemplary embodiments disclosed herein are only for illustrative purposes and should not be construed as limiting the scope of the present application. On the contrary, the present application is intended to cover not only the exemplary embodiments but also various alternatives, modifications, equivalents, and other embodiments that may be included within the spirit and scope of the present application as defined by the appended claims.

## Claims

1. A modified polypeptide having glucanase activity, in which any one or more among the amino acids corresponding to positions 53, 199, and 200 from the N-terminus of SEQ ID NO: 1 is substituted with another amino acid.

2. The modified polypeptide of claim 1, wherein the modified polypeptide comprises any one or more substitutions from the following:
i) substitution of the amino acid corresponding to position 53 with cysteine (C);
ii) substitution of the amino acid corresponding to position 199 with cysteine (C); and
iii) substitution of the amino acid corresponding to position 200 with proline (P).

3. The modified polypeptide of claim 1, wherein the modified polypeptide comprises substitutions selected from the following:
(i) substitution of the amino acid corresponding to position 200 with proline (P);
(ii) substitution of the amino acid corresponding to position 53 with cysteine (C) and substitution of the amino acid corresponding to position 199 with cysteine (C); or
(iii) substitution of the amino acid corresponding to position 53 with cysteine (C), substitution of the amino acid corresponding to position 199 with cysteine (C) and substitution of the amino acid corresponding to position 200 with proline (P).

4. The modified polypeptide of claim 1, wherein the modified polypeptide comprises substitution of the amino acid corresponding to position 53 with cysteine (C) and substitution of the amino acid corresponding to position 199 with cysteine (C), and modification of the amino acid pair to form a disulfide bond.

5. The modified polypeptide of to claim 1, wherein the modified polypeptide comprises any one amino acid sequence selected from SEQ ID NOS: 5 to 7.

6. The modified polypeptide of to claim 1, wherein the modified polypeptide has improved thermal tolerance compared to the polypeptide of SEQ ID NO: 1.

7. A polynucleotide encoding the modified polypeptide of any one of claims 1 to 6.

8. A microorganism comprising one or more among the modified polypeptide of any one of claims 1 to 6; a polynucleotide encoding the modified polypeptide; and a vector containing the polynucleotide.

9. A composition for degrading glucan, comprising one or more among the modified polypeptide of any one of claims 1 to 6; and a microorganism comprising one or more among the modified polypeptide of any one of claims 1 to 6, a polynucleotide encoding the modified polypeptide, and a vector containing the polynucleotide.

10. A method for degrading glucan, comprising: reacting one or more among the modified polypeptide of any one of claims 1 to 6; and a microorganism comprising one or more among the modified polypeptide of any one of claims 1 to 6, a polynucleotide encoding the modified polypeptide, and a vector containing the polynucleotide with glucan.

11. A composition comprising one or more among the modified polypeptide of any one of claims 1 to 6; and a microorganism comprising one or more among the modified polypeptide of any one of claims 1 to 6, a polynucleotide encoding the modified polypeptide, and a vector containing the polynucleotide.

12. A method for preparing feed products, comprising: mixing a feed additive composition comprising one or more among the modified polypeptide of any one of claims 1 to 6; and a microorganism comprising one or more among the modified polypeptide of any one of claims 1 to 6, a polynucleotide encoding the modified polypeptide, and a vector containing the polynucleotide with a feed component.

13. The composition according to claim 11, wherein the composition is for use in food, feed additive, food preparation, detergent, fabric or paper processing.

14. A method for preparing a polypeptide having glucanase activity, comprising culturing a microorganism comprising one or more among the modified polypeptide of any one of claims 1 to 6; a polynucleotide encoding the modified polypeptide; and a vector containing the polynucleotide.
